# EUROPEAN PATENT APPLICATION

(11) **EP 1 557 419 A1**
(43) Date of publication of application: **27.07.2005**
(21) Application number: 03738573.9
(22) Date of filing: 30.06.2003
(51) Int. Cl.: C07D 495/04

(54) **PYRROLE DERIVATIVE AND PROCESS FOR PRODUCING THE SAME**

(30) Priority: 05.07.2002 JP 2002197401
(71) Applicant: NIPPON SODA CO., LTD., Chiyoda-ku, Tokyo 100-8165 (JP)
(72) Inventor: KATO, Masahiko c/o Nihongi P. Nippon Soda Co.Ltd., Nakakubiki-gun, Niigata, 949-2392 (JP); KANEKO, Akira c/o Nihongi P. Nippon Soda Co. Ltd., Nakakubiki-gu, Niigata 949-2392 (JP)
(74) Representative: Wibbelmann, Jobst, Dr., Dipl.-Chem.
(86) International application number: PCT/JP2003/008266
(87) International publication number: WO 2004/005297

(57) **Abstract**

An object of the present invention is to provide novel pyrrole derivatives and processes for producing the derivatives, and to provide novel pyrrole derivatives (intermediates) which may be used as a starting material for the pyrrole derivatives and processes for producing the derivatives (intermediates). The pyrrole derivatives may be **characterized by** being represented by the formulae [1] and [2] 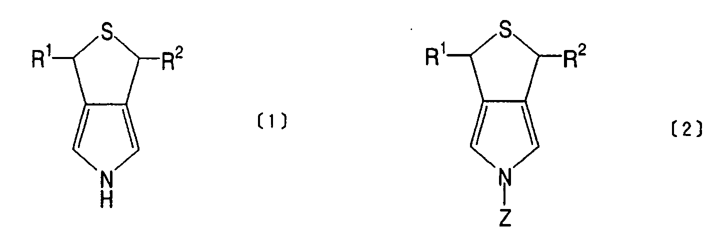 wherein R¹ and R² each independently represents a hydrogen atom or a substituted or unsubstituted C₁₋₁₀ hydrocarbon group and Z represents an organic group; and the process for producing the compound represented by the formula [1] may be **characterized by** separating Z from the compound represented by the formula [2].

## Description

### Technical Field

The present invention relates to novel pyrrole derivatives including a sulfide bond within the molecule thereof, and to processes for producing the pyrrole derivatives.

### Background Art

Recently, as high frequency waves are increasingly used for power circuits of electronic devices, demands have been increased for electrolytic capacitors used therein which have excellent high-frequency characteristics. Accordingly, in order to realize low impedance at a high frequency region, solid capacitors which utilize conducting polymers having high electric conductivity, such as polythiophene, polypyrrole, polyaniline and polyindole, as a solid electrolyte have been proposed (refer to Japanese Unexamined Patent Application, First Publication No. Sho 60-37114; Japanese Unexamined Patent Application, First Publication No. Sho 63-158829; Japanese Unexamined Patent Application, First Publication No. Hei 2-153516, and so forth).

### Disclosure of Invention

However, there is a problem in that satisfactory performance may not be obtained using the conducting polymers having conventional structure. Accordingly, an object of the present invention is to provide a compound which may be a starting material for a conducting polymer having a novel structure from which satisfactory performance may be obtained.

The inventors of the present invention have paid particular attention to compounds having a pyrrole structure and a sulfide bond within the same molecule as a compound capable of being used as a capacitor, and they have pursued diligent research. As a result, the inventors of the present invention have succeeded in synthesizing a novel pyrrole derivative having a sulfide bond under special conditions and have completed the present invention.

That is, the present invention relates to a pyrrole derivative (claim 1) represented by the formula [1]: 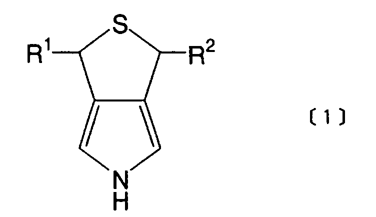 (wherein R¹ and R² each independently represents a hydrogen atom or a substituted or unsubstituted C₁₋₁₀ hydrocarbon group); to a pyrrole derivative (claim 2) represented by the formula [2]: 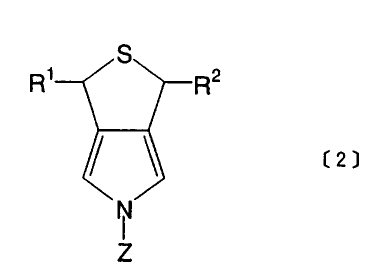 (wherein R¹ and R² each independently represents a hydrogen atom or a substituted or unsubstituted C₁₋₁₀ hydrocarbon group, and Z represents an organic group); to a pyrrole derivative (claim 3) claimed in claim 2 wherein Z is a protecting group for the nitrogen atom; and to a pyrrole derivative (claim 4) claimed in claim 3 wherein the protecting group for the nitrogen atom is a tosyl group.

Also, the present invention relates to a process for producing the pyrrole derivative (claim 5) represented by the formula [2]: 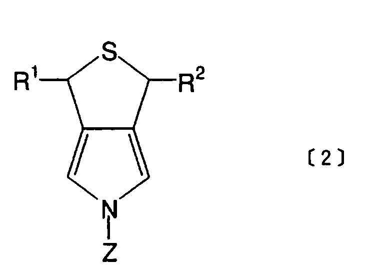 (wherein R¹ and R² each independently represents a hydrogen atom or a substituted or unsubstituted C₁₋₁₀ hydrocarbon group, and Z represents an organic group) including the step of reacting a pyrrole derivative represented by the formula [3] with an alkali metal sulfide: 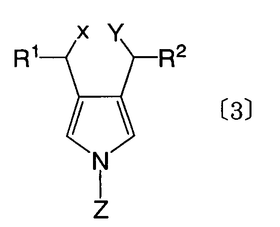 (wherein R¹ and R² each independently represents a hydrogen atom or a substituted or unsubstituted C₁₋₁₀ hydrocarbon group, X and Y each independently represents a halogen atom, and Z represents an organic group); to a process for producing the pyrrole derivative (claim 6) represented by the formula [1]: 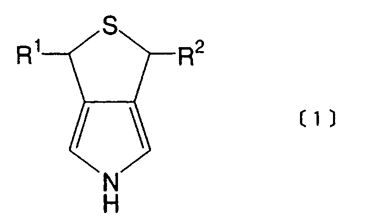 (wherein R¹ and R² each independently represents a hydrogen atom or a substituted or unsubstituted C₁₋₁₀ hydrocarbon group) including the step of deprotecting a protecting group of a pyrrole derivative represented by the formula [4]: 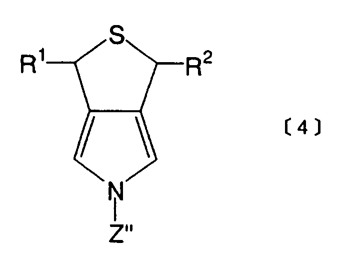 (wherein R¹ and R² each independently represents a hydrogen atom or a substituted or unsubstituted C₁₋₁₀ hydrocarbon group, and Z" represents a protecting group for the nitrogen atom); and to a process for producing the pyrrole derivative (claim 7) claimed in claim 6 wherein Z" in the formula [4] is a tosyl group and the deprotection of the tosyl group is carried out by using sodium bis(2-methoxyethoxy)aluminum hydride.

The pyrrole derivatives of the present invention may be characterized by being represented by the formula [1]. The pyrrole derivatives represented by the formula [1] may be polymerized by electrolytic polymerization, oxidation polymerization, etc., and utilized as a solid electrolyte for a solid electrolytic capacitor.

In the formula [1], R¹ and R² each independently represents a hydrogen atom or a substituted or unsubstituted C₁₋₁₀ hydrocarbon group. The hydrocarbon group represented by R¹ and R² may be a straight chain or branched group, and may be substituted with an alkoxy group, such as methoxy group and ethoxy group, an acyl group, such as formyl group and acetyl group, and so forth. The number of carbon atoms of the hydrocarbon group represented by R¹ and R² is not particularly limited as long as it is between 1 and 10. Specific examples thereof include C₁₋₁₀ alkyl groups, such as methyl group, ethyl group, n-propyl group, isopropyl group, n-butyl group, t-butyl group, s-butyl group, isobutyl group, n-pentyl group, s-pentyl group, isopentyl group, neopentyl group, n-hexyl group, s-hexyl group, 1,1-dimethyl-n-hexyl group, n-heptyl group, and n-decyl group; C₂₋₁₀ alkenyl groups, such as vinyl group, allyl group, 2-butenyl group, 1-methyl-2-propenyl group, and 4-octenyl group; C₂₋₁₀ alkynyl groups, such as ethynyl group, propargyl group, and 1-methyl-propynyl group; C₃₋₁₀ alicyclic hydrocarbon groups, such as cyclopropyl group, cyclobutyl group, cyclopentyl group, 1-methyl-cyclopentyl group, 1-methyl-cyclohexyl group, and norbornyl group; C₆₋₁₀ aromatic hydrocarbon groups, such as phenyl group, and 1-naphthyl group; and aralkyl groups, such as benzyl group, and phenethyl group. More specifically, examples of the pyrrole derivatives represented by the formula [1] include those shown in Table 1 below:

**Table 1**

| Compound No | R¹(R²) | R²(R¹) |
|---|---|---|
| 1 | CH₃ | CH₃ |
| 2 | CH₃ | CH₂CH₃ |
| 3 | CH₃ | CH₂CH₂CH₃ |
| 4 | CH₃ | CH₂CH₂CH₂CH₃ |
| 5 | CH₃ | CH₂CH₂CH₂CH₂CH₃ |
| 6 | CH₃ | CH(CH₃)₂ |
| 7 | CH₃ | CH₂CH(CH₃)₂ |
| 8 | CH₃ | CH(CH₃)CH₂CH₃ |
| 9 | CH₃ | C(CH₃)₃ |
| 10 | CH₃ | CH₂CH(CH₃)CH₂CH₃ |
| 11 | CH₃ | CH₂C(CH₃)₃ |
| 12 | CH₃ | CH₂CH₂CH(CH₃)₂ |
| 13 | CH₃ | CH(CH₃)CH₂CH₂CH₃ |
| 14 | CH₃ | CH(CH₂CH₃)₂ |
| 15 | CH₃ | CH(CH₃)CH(CH₃)₂ |
| 16 | CH₃ | CH(CH₃)₂CH₂CH₃ |
| 17 | CH₂CH₃ | CH₂CH₃ |
| 18 | CH₂CH₃ | CH₂CH₂CH₃ |
| 19 | CH₂CH₃ | CH₂CH₂CH₂CH₃ |
| 20 | CH₂CH₃ | CH₂CH₂CH₂CH₂CH₃ |
| 21 | CH₂CH₃ | CH(CH₃)₂ |
| 22 | CH₂CH₃ | CH₂CH(CH₃)₂ |
| 23 | CH₂CH₃ | CH(CH₃)CH₂CH₃ |
| 24 | CH₂CH₃ | C(CH₃)₃ |
| 25 | CH₂CH₃ | CH₂CH(CH₃)CH₂CH₃ |
| 26 | CH₂CH₃ | CH₂C(CH₃)₃ |
| 27 | CH₂CH₃ | CH₂CH₂CH(CH₃)₂ |
| 28 | CH₂CH₃ | CH(CH₃)CH₂CH₂CH₃ |
| 29 | CH₂CH₃ | CH(CH₂CH₃)₂ |
| 30 | CH₂CH₃ | CH(CH₃)CH(CH₃)₂ |
| 31 | CH₂CH₃ | CH(CH₃)₂CH₂CH₃ |
| 32 | CH₂CH₂CH₃ | CH₂CH₂CH₃ |
| 33 | CH₂CH₂CH₃ | CH₂CH₂CH₂CH₃ |
| 34 | CH₂CH₂CH₃ | CH₂CH₂CH₂CH₂CH₃ |
| 35 | CH₂CH₂CH₃ | CH(CH₃)₂ |
| 36 | CH₂CH₂CH₃ | CH₂CH(CH₃)₂ |
| 37 | CH₂CH₂CH₃ | CH(CH₃)CH₂CH₃ |
| 38 | CH₂CH₂CH₃ | C(CH₃)₃ |

| | | |
|---|---|---|
| 39 | CH₂CH₂CH₃ | CH₂CH(CH₃)CH₂CH₃ |
| 40 | CH₂CH₂CH₃ | CH₂C(CH₃)₃ |
| 41 | CH₂CH₂CH₃ | CH₂CH₂CH(CH₃)₂ |
| 42 | CH₂CH₂CH₃ | CH(CH₃)CH₂CH₂CH₃ |
| 43 | CH₂CH₂CH₃ | CH(CH₂CH₃)₂ |
| 44 | CH₂CH₂CH₃ | CH(CH₃)CH(CH₃)₂ |
| 45 | CH₂CH₂CH₃ | CH(CH₃)₂CH₂CH₃ |
| 46 | CH₂CH₂CH₂CH₃ | CH₂CH₂CH₂CH₃ |
| 47 | CH₂CH₂CH₂CH₃ | CH₂CH₂CH₂CH₂CH₃ |
| 48 | CH₂CH₂CH₂CH₃ | CH(CH₃)₂ |
| 49 | CH₂CH₂CH₂CH₃ | CH₂CH(CH₃)₂ |
| 50 | CH₂CH₂CH₂CH₃ | CH(CH₃)CH₂CH₃ |
| 51 | CH₂CH₂CH₂CH₃ | C(CH₃)₃ |
| 52 | CH₂CH₂CH₂CH₃ | CH₂CH(CH₃)CH₂CH₃ |
| 53 | CH₂CH₂CH₂CH₃ | CH₂C(CH₃)₃ |
| 54 | CH₂CH₂CH₂CH₃ | CH₂CH₂CH(CH₃)₂ |
| 55 | CH₂CH₂CH₂CH₃ | CH(CH₃)CH₂CH₂CH₃ |
| 56 | CH₂CH₂CH₂CH₃ | CH(CH₂CH₃)₂ |
| 57 | CH₂CH₂CH₂CH₃ | CH(CH₃)CH(CH₃)₂ |
| 58 | CH₂CH₂CH₂CH₃ | CH(CH₃)₂CH₂CH₃ |
| 59 | CH₂CH₂CH₂CH₂CH₃ | CH₂CH₂CH₂CH₂CH₃ |
| 60 | CH₂CH₂CH₂CH₂CH₃ | CH(CH₃)₂ |
| 61 | CH₂CH₂CH₂CH₂CH₃ | CH₂CH(CH₃)₂ |
| 62 | CH₂CH₂CH₂CH₂CH₃ | CH(CH₃)CH₂CH₃ |
| 63 | CH₂CH₂CH₂CH₂CH₃ | C(CH₃)₃ |
| 64 | CH₂CH₂CH₂CH₂CH₃ | CH₂CH(CH₃)CH₂CH₃ |
| 65 | CH₂CH₂CH₂CH₂CH₃ | CH₂C(CH₃)₃ |
| 66 | CH₂CH₂CH₂CH₂CH₃ | CH₂CH₂CH(CH₃)₂ |
| 67 | CH₂CH₂CH₂CH₂CH₃ | CH(CH₃)CH₂CH₂CH₃ |
| 68 | CH₂CH₂CH₂CH₂CH₃ | CH(CH₂CH₃)₂ |
| 69 | CH₂CH₂CH₂CH₂CH₃ | CH(CH₃)CH(CH₃)₂ |
| 70 | CH₂CH₂CH2CH₂CH₃ | CH(CH₃)₂CH₂CH₃ |
| 71 | CH(CH₃)₂ | CH(CH₃)₂ |
| 72 | CH(CH₃)₂ | CH₂CH(CH₃)₂ |
| 73 | CH(CH₃)₂ | CH(CH₃)CH₂CH₃ |
| 74 | CH(CH₃)₂ | C(CH₃)₃ |
| 75 | CH(CH₃)₂ | CH₂CH(CH₃)CH₂CH₃ |
| 76 | CH(CH₃)₂ | CH₂C(CH₃)₃ |
| 77 | CH(CH₃)₂ | CH₂CH₂CH(CH₃)₂ |

| | | |
|---|---|---|
| 78 | CH(CH₃)₂ | CH(CH₃)CH₂CH₂CH₃ |
| 79 | CH(CH₃)₂ | CH(CH₂CH₃)₂ |
| 80 | CH(CH₃)₂ | CH(CH₃)CH(CH₃)₂ |
| 81 | CH(CH₃)₂ | CH(CH₃)₂CH₂CH₃ |
| 82 | CH₂CH(CH₃)₂ | CH₂CH(CH₃)₂ |
| 83 | CH₂CH(CH₃)₂ | CH(CH₃)CH₂CH₃ |
| 84 | CH₂CH(CH₃)₂ | C(CH₃)₃ |
| 85 | CH₂CH(CH₃)₂ | CH₂CH(CH₃)CH₂CH₃ |
| 86 | CH₂CH(CH₃)₂ | CH₂C(CH₃)₃ |
| 87 | CH₂CH(CH₃)₂ | CH₂CH₂CH(CH₃)₂ |
| 88 | CH₂CH(CH₃)₂ | CH(CH₃)CH₂CH₂CH₃ |
| 89 | CH₂CH(CH₃)₂ | CH(CH₂CH₃)₂ |
| 90 | CH₂CH(CH₃)₂ | CH(CH₃)CH(CH₃)₂ |
| 91 | CH₂CH(CH₃)₂ | CH(CH₃)₂CH₂CH₃ |
| 92 | CH(CH₃)CH₂CH₃ | CH(CH₃)CH₂CH₃ |
| 93 | CH(CH₃)CH₂CH₃ | C(CH₃)₃ |
| 94 | CH(CH₃)CH₂CH₃ | CH₂CH(CH₃)CH₂CH₃ |
| 95 | CH(CH₃)CH₂CH₃ | CH₂C(CH₃)₃ |
| 96 | CH(CH₃)CH₂CH₃ | CH₂CH₂CH(CH₃)₂ |
| 97 | CH(CH₃)CH₂CH₃ | CH(CH₃)CH₂CH₂CH₃ |
| 98 | CH(CH₃)CH₂CH₃ | CH(CH₂CH₃)₂ |
| 99 | CH(CH₃)CH₂CH₃ | CH(CH₃)CH(CH₃)₂ |
| 100 | CH(CH₃)CH₂CH₃ | CH(CH₃)₂CH₂CH₃ |
| 101 | C(CH₃)₃ | C(CH₃)₃ |
| 102 | C(CH₃)₃ | CH₂CH(CH₃)CH₂CH₃ |
| 103 | C(CH₃)₃ | CH₂C(CH₃)₃ |
| 104 | C(CH₃)₃ | CH₂CH₂CH(CH₃)₂ |
| 105 | C(CH₃)₃ | CH(CH₃)CH₂CH₂CH₃ |
| 106 | C(CH₃)₃ | CH(CH₂CH₃)₂ |
| 107 | C(CH₃)₃ | CH(CH₃)CH(CH₃)₂ |
| 108 | C(CH₃)₃ | CH(CH₃)₂CH₂CH₃ |
| 109 | CH₂CH(CH₃)CH₂CH₃ | CH₂CH(CH₃)CH₂CH₃ |
| 110 | CH₂CH(CH₃)CH₂CH₃ | CH₂C(CH₃)₃ |
| 111 | CH₂CH(CH₃)CH₂CH₃ | CH₂CH₂CH(CH₃)₂ |
| 112 | CH₂CH(CH₃)CH₂CH₃ | CH(CH₃)CH₂CH₂CH₃ |
| 113 | CH₂CH(CH₃)CH₂CH₃ | CH(CH₂CH₃)₂ |
| 114 | CH₂CH(CH₃)CH₂CH₃ | CH(CH₃)CH(CH₃)₂ |
| 115 | CH₂CH(CH₃)CH₂CH₃ | CH(CH₃)₂CH₂CH₃ |
| 116 | CH₂C(CH₃)₃ | CH₂C(CH₃)₃ |

| | | |
|---|---|---|
| 117 | CH₂C(CH₃)₃ | CH₂CH₂CH(CH₃)₂ |
| 118 | CH₂C(CH₃)₃ | CH(CH₃)CH₂CH₂CH₃ |
| 119 | CH₂C(CH₃)₃ | CH(CH₂CH₃)₂ |
| 120 | CH₂C(CH₃)₃ | CH(CH₃)CH(CH₃)₂ |
| 121 | CH₂C(CH₃)₃ | CH(CH₃)₂CH₂CH₃ |
| 122 | CH₂CH₂CH (CH₃)₂ | CH₂CH₂CH(CH₃)₂ |
| 123 | CH₂CH₂CH (CH₃)₂ | CH(CH₃)CH₂CH₂CH₃ |
| 124 | CH₂CH₂CH(CH₃)₂ | CH(CH₂CH₃)₂ |
| 125 | CH₂CH₂CH(CH₃)₂ | CH(CH₃)CH(CH₃)₂ |
| 126 | CH₂CH₂CH(CH₃)₂ | CH(CH₃)₂CH₂CH₃ |
| 127 | CH(CH₃)CH₂CH₂CH₃ | CH(CH₃)CH₂CH₂CH₃ |
| 128 | CH(CH₃)CH₂CH₂CH₃ | CH(CH₂CH₃)₂ |
| 129 | CH(CH₃)CH₂CH₂CH₃ | CH(CH₃)CH(CH₃)₂ |
| 130 | CH(CH₃)CH₂CH₂CH₃ | CH(CH₃)₂CH₂CH₃ |
| 131 | CH(CH₂CH₃)₂ | CH(CH₂CH₃)₂ |
| 132 | CH(CH₂CH₃)₂ | CH(CH₃)CH(CH₃)₂ |
| 133 | CH(CH₂CH₃)₂ | CH(CH₃)₂CH₂CH₃ |
| 134 | CH(CH₃)CH(CH₃)₂ | CH(CH₃)CH(CH₃)₂ |
| 135 | CH(CH₃)CH(CH₃)₂ | CH(CH₃)₂CH₂CH₃ |
| 136 | CH(CH₃)₂CH₂CH₃ | CH(CH₃)₂CH₂CH₃ |

Also, the pyrrole derivatives according to the present invention may be characterized by being represented by the formula [2]. The pyrrole derivatives represented by the formula [2] are compounds which may be an intermediate in the process of producing the pyrrole derivatives represented by the formula [1].

In the formula [2], R¹ and R² each independently represents a hydrogen atom or a substituted or unsubstituted C₁₋₁₀ hydrocarbon group, and Z represents an organic group (i.e., an organic functional group). The definition of R¹ and R², respectively, in the formula [2] is the same as that of R¹ and R², respectively, in the formula [1]. Also, the above-mentioned organic group is not particularly limited as long as it contains a carbon atom in the functional group. Accordingly, it is not necessary for a carbon atom to be directly bonded to the nitrogen atom in the pyrrole ring, and examples thereof include functional groups in which a carbon atom is bonded via a hetero atom, etc., such as a nitrogen atom, sulfur atom, oxygen atom, and phosphorus atom. In particular, a protecting group which is capable of protecting the nitrogen atom at the pyrrole portion is preferable. Specific examples thereof include alkoxy carbonyl group, aryl sulfonyl group, alkyloxymethyl group, trialkylsilyl group (these may include a substituent, such as alkyl group, alkoxy group, and acyl group). Among these, the aryl sulfonyl group is preferable, and a tosyl group (p-toluene sulfonyl group) is particularly preferable.

As a process for producing the pyrrole derivatives represented by the formula [2], specific examples thereof include a process in which the pyrrole derivative represented by the formula [3] is reacted with an alkali metal sulfide. Also, as a process for producing the pyrrole derivatives represented by the formula [1], specific examples thereof include a process in which the protecting group of the pyrrole derivative represented by the formula [4] is deprotected.

The definition of R¹ and R², respectively, in the formula [3] is the same as that of R¹ and R², respectively, in the formula [1]. Also, in the formula [3], examples of the halogen atom represented by X and Y include fluorine atom, chlorine atom, bromine atom, and iodine atom. Among these, chlorine atom and bromine atom are preferable. Moreover, depending on the situation, X and Y may be aryl sulfonyloxy group, alkyl sulfonyloxy group, or trifluoromethylsulfonyloxy group. The definition of Z in the formula [3] is the same as that of Z in the formula [2].

In the formula [4], R¹ and R² each independently represents a hydrogen atom or a substituted or unsubstituted C₁₋₁₀ hydrocarbon group, and Z" represents a protecting group for the nitrogen atom. The definition of R¹ and R², respectively, in the formula [4] is the same as that of R¹ and R², respectively, in the formula [1]. Also, the definition of Z" in the formula [4] is the same as that of Z which is a protecting group for the nitrogen atom in the formula [2].

Examples of solvent which may be suitably used in the above-mentioned reaction include water, C₁₋₆ straight chain or branched alcohols, such as methanol, ethanol, propanol, and ethyleneglycol; straight chain or cyclic ether, such as tetrahydrofuran, diethylether, dioxane, glyme, and diglyme; chlorine containing solvents, such as chloroform, dichloromethane, chlorobenzene, and dichlorobenzene; and aprotic polar solvents, such as dimethyl formamide, and dimethylsulfoxide, and these may be used alone or in a mixture of two or more.

The reaction temperature is preferably within a range between -10°C and the boiling point of the solvent used. If the temperature is within the above-mentioned range, the reaction may proceed more smoothly.

Examples of the above-mentioned alkali metal sulfide include potassium sulfide and sodium sulfide, and it is possible to use hydrate and anhydride thereof prepared in advance. Also, it is possible to directly use one which is purified in a reaction system of alkali metal alcoholate and hydrogen sulfide. The amount of alkali metal sulfide is 1 to 100 equivalents, preferably 1.0 to 2.0 equivalents, with respect to the pyrrole derivative represented by the formula [3].

The pyrrole derivatives represented by the formula [1] may be produced by deprotecting the protecting group of the pyrrole derivative represented by the formula [4]. This deprotection process may be carried out after isolating the pyrrole derivative represented by the formula [4] or without isolating the pyrrole derivative.

The deprotection process of the protecting group (Z") may be performed by, for example; hydrolysis using mineral acids, such as hydrochloric acid and sulfuric acid, or alkali, such as sodium hydroxide, potassium hydroxide, sodium sulfide, and sodium disulfide; alcoholysis using metal alcoholate; or a reduction reaction using lithium aluminum hydride, sodium borohydride, sodium bis(2-methoxyethoxy) aluminum hydride ("Red-A1" (registered trademark)). Among these, the reduction reaction is preferable and it is particularly preferable to use sodium bis(2-methoxyethoxy) aluminum hydride when a deprotection process for tosyl group, which is a protecting group, is performed. The amount of a reduction agent added is preferably within the range of 1 to 4 equivalents, more preferably 1.5 to 3 equivalents, with respect to the pyrrole derivative represented by the formula [4].

The pyrrole derivative which is produced as explained above, may be polymerized by electrolytic polymerization, oxidation polymerization etc., and utilized as a solid electrolyte (electrode) for a solid electrolytic capacitor.

### Best Mode for Carrying Out the Invention

Hereinafter, the present invention will be explained in detail with reference to an example; however, it is apparent that the technical scope of the present invention is not limited by the example by any means.

### Example 1 <Synthesis of 3,5-dihydro-1H-thieno-[3,4-c] pyrrole>

Dichlorotriphenyl phosphoran (6.44 g, 19.3 mmol) was suspended in 30 ml of tetrahydrofuran (THF), and 3,4-di(hydroxymethyl)-1-tosylpyrrole (2.47 g, 8.8 mmol) was slowly added thereto while being cooled by ice water. After the mixture was stirred for two hours at room temperature, the solvent of THF was evaporated under reduced pressure, and then water and ethyl acetate were added to separate layers. The layer of ethyl acetate was dried over magnesium sulfate anhydride, and was concentrated under reduced pressure. After purification using column chromatography ("Wako Gel C200"; hexane : ethyl acetate = 8 : 2 (slurry was used), 2.55 g of 3,4-di(chloromethyl)-1-tosylpyrrole was obtained (yield of 91 %).
3,4-di(chloromethyl)-1-tosylpyrrole:
¹H-NMR (270MHz, CDCl₃/TMS) d 2.43 (s, 3H), 4.53 (s, 4H), 7.17 (s, 2H), 7.33 (d, 2H, J = 8.1Hz), 7.77 (d, 2H, J = 8.1Hz)

After 2.55 g (8 mmol) of 3,4-di(chloromethyl)-1-tosylpyrrole was dissolved in 20 ml of methanol, 2.12 g (8.8 mmol) of sodium sulfide 9 hydrate (Na₂S·9H₂O) was added and the mixture was heated under reflux for 3 hours. After being cooled, water and ethyl acetate were added, and the mixture was subjected to cerite filtration. The filtrate was separated and the organic layer was washed with saline, dried over magnesium sulfate anhydride, concentrated, and purified using column chromatography ("Wako Gel C200"; hexane : ethyl acetate = 95 : 5). As a result, 0. 99 g of N-tosyl-3,5-dihydro-1H-thieno-[3,4-c] pyrrole was obtained (yield of 44%).
N-tosyl-3,5-dihydro-1H-thieno-[3,4-c] pyrrole: crystal, mp 134-136°C

After this, 0.99 g of N-tosyl-3,5-dihydro-1H-thieno-[3,4-c] pyrrole was dissolved in 15 ml of benzene, and Red-A1 (registered trademark) (65% by weight) 2.20 g (2 equivalents) was added, and the mixture was heated under reflux for 2 hours. After being cooled, ice was added to eliminate excess reduction agent, and then water and ethyl acetate were added to separate layers. The organic layer was washed with saline, dried over magnesium sulfate anhydride, concentrated, and purified using column chromatography ("Wako Gel C200"; hexane : ethyl acetate = 95 : 5). As a result, 0. 23 g of 3,5-dihydro-1H-thieno-[3,4-c] pyrrole was obtained (yield of 52%).
3,5-dihydro-1H-thieno-[3,4-c] pyrrole
¹H-NMR (270MHz, CDCl₃/TMS) d = 4.00 (s, 4H), 6.48 (d, 2H, J = 2.4Hz), 8.15 (bs, 1H), crystal, mp 39-40°C

### Industrial Applicability

As described above, the pyrrole derivatives of the present invention are novel compounds which may be a starting material for a conducting polymer that can be used as an electrolyte of a solid electrolytic capacitor, and hence the industrial applicability thereof is high.

## Claims

1. A pyrrole derivative represented by a formula [1]: 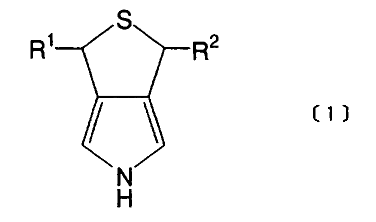 (wherein R¹ and R² each independently represents a hydrogen atom or a substituted or unsubstituted C₁₋₁₀ hydrocarbon group).

2. A pyrrole derivative represented by a formula [2]: 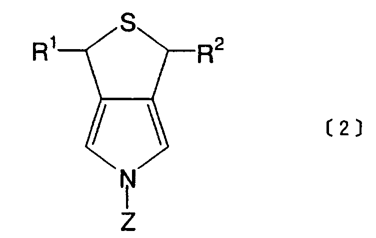 (wherein R¹ and R² each independently represents a hydrogen atom or a substituted or unsubstituted C₁₋₁₀ hydrocarbon group, and Z represents an organic group).

3. A pyrrole derivative according to claim 2, wherein Z is a protecting group for the nitrogen atom.

4. A pyrrole derivative according claim 3, wherein the protecting group for the nitrogen atom is a tosyl group.

5. A process for producing the pyrrole derivative represented by the formula [2]: 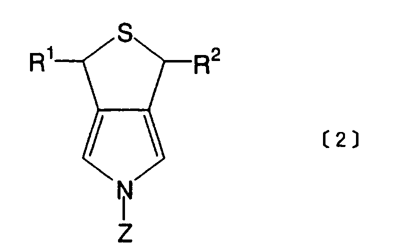 (wherein R¹ and R² each independently represents a hydrogen atom or a substituted or unsubstituted C₁₋₁₀ hydrocarbon group, and Z represents an organic group), comprising the step of reacting a pyrrole derivative represented by a formula [3] with an alkali metal sulfide: 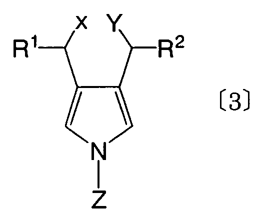 (wherein R¹ and R² each independently represents a hydrogen atom or a substituted or unsubstituted C₁₋₁₀ hydrocarbon group, X and Y each independently represents a halogen atom, and Z represents an organic group).

6. A process for producing the pyrrole derivative represented by the formula [1]: 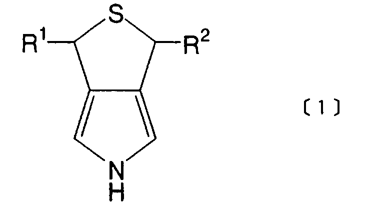 (wherein R¹ and R² each independently represents a hydrogen atom or a substituted or unsubstituted C₁₋₁₀ hydrocarbon group), comprising the step of deprotecting a protecting group of a pyrrole derivative represented by a formula [4]: 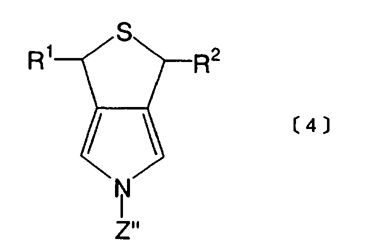 (wherein R¹ and R² each independently represents a hydrogen atom or a substituted or unsubstituted C₁₋₁₀ hydrocarbon group, and Z" represents the protecting group for the nitrogen atom).

7. A process for producing the pyrrole derivative according to claim 6, wherein Z" in the formula [4] is a tosyl group and the deprotection of the tosyl group is carried out by using sodium bis(2-methoxyethoxy) aluminum hydride.
